# FASCICULE DE BREVET EUROPEEN

(11) **EP 1 688 128 B1**
(45) Date de publication et mention de la délivrance du brevet: **07.03.2018**
(21) Numéro de dépôt: 06290131.9
(22) Date de dépôt: 19.01.2006
(51) Int. Cl.: A61K 8/60, A61K 8/67, A61Q 7/00, A61Q 5/12

(54) **Procédé de traitement des fibres kératiniques et du cuir chevelu avec une composition contenant un dérivé de glucose et de vitamine F**
Verfahren zur Behandlung keratinhaltiger Fasern und der Kopfhaut mit einer Zusammensetzung enthaltend ein Glucose und Vitamin F Derivat
Method for treating keratinic fibres and the scalp with a composition containing a derivative of glucose and vitamin F

(30) Priorité: 08.02.2005 FR 0550363
(43) Date de publication de la demande: 09.08.2006
(73) Titulaire: L'Oréal, 75008 Paris (FR)
(72) Inventeur: Mezure, Patricia, 78380 Bougival (FR); Dubief, Claude, 78150 Le Chesnay (FR)
(74) Mandataire: Miszputen, Laurent

(56) Documents cités:
- EP-A- 0 491 532
- EP-A- 1 371 658
- WO-A-93/02657
- WO-A2-2004/034958
- US-A- 5 281 420
- US-A1- 2006 013 787
- Khitam Al-Refu: "Scalp Biopsy and Diagnosis of Common Hair Loss Problems" In: "Skin Biopsy - Diagnosis and Treatment", 3 July 2013 (2013-07-03), InTech, XP055117193, ISBN: 978-9-53-511173-3 DOI: 10.5772/55025,
- Anonymous: "RÖMPP Online - ID=RD-20-00650, Tenside", , 12 May 2014 (2014-05-12), XP055117473, Retrieved from the Internet: URL:http://www.roempp.com/prod3/roempp.php [retrieved on 2014-05-12]

## Description

### DOMAINE DE L'INVENTION

L'invention a pour objet un procédé de traitement non thérapeutique cosmétique des fibres kératiniques humaines et/ou du cuir chevelu consistant à appliquer une composition physiologiquement acceptable contenant, entre autre, un dérivé de glucose et de vitamine F et un tensioactif spécifique, destinée à améliorer l'état des fibres kératiniques humaines et notamment à diminuer ou à freiner leur chute et/ou à induire et/ou à stimuler leur croissance.

Les fibres kératiniques humaines auxquelles s'applique l'invention sont notamment les cheveux, les sourcils, les cils, les poils de barbe, de moustache et les poils pubiens. Plus spécialement, l'invention s'applique aux cheveux et/ou aux cils humains.

Aussi, de façon plus précise, l'invention met en oeuvre une composition physiologiquement acceptable qui contient un dérivé de glucose et de vitamine F et un tensioactif non ionique particulier. Elle est destinée à améliorer l'état des cheveux humains et notamment d'induire et/ou stimuler la croissance des cheveux et/ou freiner leur chute. Chez l'être humain, la croissance des cheveux et leur renouvellement sont principalement déterminés par l'activité des follicules pileux et de leur environnement dermo-épidermique. Leur activité est cyclique et comporte essentiellement trois phases, à savoir la phase anagène, la phase catagène et la phase télogène.
A la phase anagène (phase active ou phase de croissance), qui dure plusieurs années et au cours de laquelle les cheveux s'allongent, succède une phase catagène très courte et transitoire qui dure quelques semaines. Au cours de cette phase, le cheveu subit une évolution, le follicule s'atrophie et son implantation dermique apparaît de plus en plus haute.
La phase terminale, qui dure quelques mois, correspond à une phase de repos, appelée phase télogène. A la fin de cette période de repos, les cheveux tombent et un autre cycle recommence.
La chevelure se renouvelle donc en permanence, et sur les 150 000 cheveux environ que comporte une chevelure, à chaque instant, 10% d'entre eux environ sont au repos et seront donc remplacés en quelques mois.
La littérature évoque de nombreuses raisons entraînant la chute précoce des cheveux ; en particulier cette chute précoce survient chez des sujets prédisposés génétiquement et elle atteint notamment les hommes. Il s'agit plus particulièrement de l'alopécie androgénétique ou androgénique ou encore androgéno-génétique.
Cette alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits « terminaux » au stade de duvets. Des zones sont touchées préférentiellement ; notamment chez l'homme, les golfes temporaux ou frontaux ainsi que la partie supérieure de l'occipital, tandis que chez les femmes on constate plutôt une alopécie diffuse du vertex.
D'autres causes peuvent entraîner une importante, temporaire ou définitive, des cheveux. Il peut s'agir de chute et d'altération des cheveux après une grossesse (post partum), au cours d'états de dénutrition ou de déséquilibres alimentaires ou encore au cours d'états d'asthénie ou de dysfonctionnement hormonal comme cela peut être le cas au cours ou après la ménopause. Il peut également s'agir de chute ou d'altérations des cheveux en relation avec des phénomènes saisonniers.

### ARRIERE PLAN DE L'INVENTION

On recherche depuis de nombreuses années, notamment dans l'industrie cosmétique, des compositions permettant de supprimer ou de réduire l'effet de l'alopécie et notamment d'induire ou de stimuler la croissance des cheveux et/ou de certains poils, voire de diminuer ou retarder leur chute.

Dans cette optique, un nouveau composé bien particulier, dérivé O-acylé du glucose, présente des propriétés remarquables justifiant son utilisation comme actif pour limiter la chute des cheveux et/ou favoriser leur repousse ; ce composé correspond au résultat de l'estérification du glucose par la vitamine F. Sa fabrication est décrite dans la demande FR-A-2 840 903 dans laquelle sont illustrées des compositions contenant notamment le dérivé de glucose et de vitamine F dans un milieu hydroalcoolique.

La formulation de ce dérivé dans une composition cosmétique ou dermatologique doit respecter un certain nombre de contraintes techniques afin qu'il puisse jouer, efficacement, son rôle d'actif anti-chute/repousse des cheveux. Ainsi, ce dérivé doit être parfaitement solubilisé dans la composition. Cette dernière, généralement utilisée sans rinçage, doit s'appliquer facilement sur la totalité de la partie du cuir chevelu à traiter, n'occasionner aucune irritation et ne pas coller. De plus, les cheveux qui peuvent être imprégnés par la composition doivent conserver un toucher agréable (ni rêche, ni gras, ni collant...), un aspect propre et gonflant.

### EXPOSE DE L'INVENTION

Après de nombreuses études, la demanderesse a découvert que ces contraintes techniques étaient résolues en formulant ce dérivé dans une composition contenant un tensioactif non ionique de balance hydrophile-lipophile (HLB) supérieure à 10 et un alcool en C₁-C₄.

La présente invention a donc pour objet un procédé de traitement cosmétique des fibres kératiniques humaines et/ou du cuir chevelu consistant à appliquer une composition physiologiquement acceptable comprenant :
(a) au moins un dérivé de glucose et de vitamine F,
(b) au moins un tensioactif non ionique de balance hydrophile/lipophile supérieure à 10,
(c) au moins un alcool en C₁-C₄, et
(d) de l'eau;
à laisser celle-ci au contact de ces fibres kératiniques et/ou du cuir chevelu, et éventuellement à rincer ces fibres kératiniques et/ou le cuir chevelu. Cette composition est en particulier destinée à induire et/ou stimuler la croissance des fibres kératiniques et/ou freiner leur chute et/ou augmenter leur densité. Elle peut être utilisée telle quelle ou ajoutée à une composition physiologiquement acceptable plus complexe.

Par augmenter la densité des fibres kératiniques, et notamment la densité capillaire, on entend augmenter le nombre de fibres kératiniques, notamment de cheveux, par cm² de peau telle que le cuir chevelu. Ainsi, cette composition permet de maintenir en bon état la chevelure et/ou lutter contre la chute naturelle des cheveux en particulier des hommes. L'utilisation cosmétique de l'association d'au moins un dérivé de glucose et de vitamine F, d'au moins un tensioactif non ionique de balance hydrophile/lipophile supérieure à 10, d'au moins un alcool en C₁-C₄ et d'eau, dans une composition notamment cosmétique de soin capillaire d'être humain, permet de traiter l'alopécie d'origine naturelle et en particulier l'alopécie androgénique ou androchrono-génétique. Le procédé de traitement présente bien les caractéristiques d'un procédé cosmétique dans la mesure où il permet d'améliorer l'esthétique des fibres kératiniques et en particulier des cheveux et des cils d'êtres humains en leur donnant une plus grande vigueur et un aspect amélioré. En outre, il peut être utilisé quotidiennement pendant plusieurs mois, sans prescription médicale.

Plus spécialement, la présente invention a pour objet un procédé de soin cosmétique des cheveux et/ou du cuir chevelu humains, en vue d'améliorer leur état et/ou leur aspect, caractérisé en ce qu'il consiste à appliquer sur les cheveux et/ou le cuir chevelu humains, une composition cosmétique telle que définie ci-dessus, à laisser celle-ci au contact des cheveux et/ou du cuir chevelu, et éventuellement à rincer les cheveux et/ou le cuir chevelu.

### DESCRIPTION DETAILLEE DES MODES DE REALISATION DE L'INVENTION

Selon l'invention, "au moins un" signifie un ou plusieurs (2, 3 ou plus). En particulier, la composition peut contenir un ou plusieurs dérivés de glucose et de vitamine F.

Dans la suite du texte, et sauf indication contraire, les quantités des différents ingrédients de la composition sont données en pourcentage en poids par rapport au poids total de la composition.

### Matières premières essentielles à l'invention

### Dérivé de glucose et de vitamine F :

Le dérivé de glucose et de vitamine F est un dérivé O-acylé obtenu par estérification, partielle ou totale, de la vitamine F par le glucose, dont la fabrication est décrite dans le document EP-A-1371658. Ce dérivé peut être représenté par un composé de formule suivante (I) ou un mélange de composés de formule (I) : 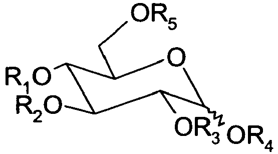 dans laquelle R₁, R₂, R₃, R₄ et R₅, indépendamment les uns des autres, représentent l'hydrogène ou un radical -CO-R avec R représentant une chaîne hydrocarbonée linéaire, saturée ou insaturée, comprenant 11 à 21 atomes de carbone,
sous réserve qu'au moins un des radicaux R₁ à R₅ soit différent de l'hydrogène.

En particulier, le dérivé de glucose et de vitamine F comprend un mélange de mono ou di- ester d'acides gras en C₁₆-C₁₈. L'acide gras est en particulier choisi parmi l'acide linoléique, l'acide oléique, l'acide palmitique, l'acide stéarique et leurs mélanges. Ce dérivé de glucose et de vitamine F est notamment choisi parmi les composés 6-O-octadeca-9-enoyl-D-glucopyranose, 6-O-hexadecanoyl-D-glucopyranose, 6-O-octadeca -9,12-dienoyl -D-glucopyranose, 6-O-octadecanoyl-D-glucopyranose, 3-O-octadeca-9,12-dienoyl -D-glucopyranose, leurs mélanges.

Le dérivé est notamment l'ester de glucose et de vitamine F, majoritairement estérifié en position 6, obtenu conformément à l'exemple 1 du document EP-A-1371658.

La quantité de dérivé de glucose et de vitamine F, utilisée, correspond à la quantité nécessaire pour obtenir le résultat désiré (en particulier à savoir augmenter la densité des fibres kératiniques ou favoriser leur pousse). L'homme du métier est donc en mesure d'évaluer cette quantité efficace qui dépend du ou des dérivés utilisés, de la personne sur laquelle on l'applique, et du temps de cette application.

Pour donner un ordre de grandeur, dans la composition selon l'invention, le dérivé de vitamine F et de glucose ou le mélanges de dérivés de vitamine F et de glucose peut être utilisé en une quantité représentant de 0,01 % à 10 % en poids, préférentiellement 0,05 % à 5 % en poids et plus préférentiellement de 0,1 % à 2 % en poids.

### Tensioactif non ionique :

La composition selon l'invention peut contenir un ou plusieurs tensioactifs non ioniques possédant une balance hydrophile-lipophile (HLB) supérieure à 10 (voir GRIFFIN, W.C. J. Soc. Cosmet. Chem. 1949, 1, 311. et 1954, 5, 249.) et pouvant aller jusqu'à 20 ; ce sont des composés bien connus en soi (voir notamment à cet égard « Handbook of Surfactants » par M. R. PORTER, éditions Blackie & Son (Glasgow and London), 1991, pp 116-178). Ainsi, ils peuvent être notamment choisis parmi les alcools, les alpha-diols, les alkyl(C₁-C₂₀)phénols ou les acides gras, polyéthoxylés, polypropoxylés ou polyglycérolés, notamment hydrogénés, ayant une chaîne grasse comportant, par exemple, de 8 à 22 atomes de carbone (notamment de 12 à 18), le nombre moyen de groupements d'oxyde d'éthylène ou d'oxyde de propylène pouvant aller notamment de 3,5 à 200 (par exemple de 5 à 100) et le nombre de groupements de glycérol pouvant aller notamment de 2 à 100 (par exemple de 3 à 50), leurs mélanges.

On peut également citer les copolymères d'oxyde d'éthylène et de propylène, les condensats d'oxyde d'éthylène et de propylène sur des alcools gras ; les amides gras polyéthoxylés et de préférence ceux ayant en moyenne de 3,5 à 200 moles d'oxyde de propylène et/ou d'éthylène (par exemple de 5 à 100) ; les amides gras polyglycérolés et de préférence ceux ayant en moyenne de 1,5 à 40 groupements de glycérol et en particulier 1,5 à 25 ; les esters d'acides gras du sorbitane éthoxylés ayant notamment de 2 à 30 moles en moyenne d'oxyde d'éthylène et une chaîne grasse ayant notamment de 8 à 22 atomes de carbone (par exemple de 12 à 18) ; les esters d'acides gras du saccharose ; les esters d'acides gras du polyéthylèneglycol ; les alkyl(C₆-C₂₄)polyglycosides ; les dérivés de N-alkyl(C₆-C₂₄)glucamine ; les oxydes d'amines tels que les oxydes d'alkyl(C₁₀-C₁₄)amines ; et leurs mélanges. Par acides gras, on entend des acides mono ou polyacides ayant de 8 à 22 atomes de carbone et mieux de 12 à 18 atomes.

Selon un mode de réalisation, on utilise comme tensioactif non ionique de HLB supérieure à 10 un ou plusieurs tensioactifs choisis parmi les alcools, les alpha-diols, les alkyl(C₁-C₂₀)phénols ou les acides gras polyéthoxylés, polypropoxylés ou polyglycérolés, hydrogénés ayant une chaîne grasse comportant de 8 à 18 atomes de carbone, le nombre moyen de groupements d'oxyde d'éthylène ou d'oxyde de propylène allant de 3,5 à 200 et le nombre de groupements de glycérol allant de 2 à 100, leurs mélanges.

Le tensioactif non-ionique ou le mélange de tensioactifs non-ioniques, de HLB supérieure à 10, est utilisé de préférence en quantité suffisante pour solubiliser avec l'alcool le dérivé de vitamine F et de glucose. Sa teneur est donc fonction de la teneur en dérivé de vitamine F et de glucose. En pratique, ce tensioactif non-ionique ou mélange de tensioactifs non-ioniques peut être utilisé dans la composition de l'invention, à une concentration allant de 0,01 % à 10 % en poids, préférentiellement de 0,05 % à 5 % en poids et plus préférentiellement de 0,1 % à 2 % en poids.

### L'alcool :

Il peut être utilisé seul ou en mélange et est choisi parmi les alcools en C₁-C₄ comme l'éthanol ou l'isopropanol, leurs mélanges. L'alcool en C₁-C₄ ou le mélange d'alcools en C₁-C₄ est utilisé de préférence en quantité suffisante pour solubiliser avec le tensioactif non-ionique le dérivé de vitamine F et de glucose. Sa teneur est donc fonction de la teneur en dérivé de vitamine F et de glucose. En pratique, l'alcool en C₁-C₄ ou le mélange d'alcools en C₁-C₄ peut être utilisé dans la composition de l'invention à une concentration allant de 2 % à 80% du poids total de la composition, préférentiellement de 10 % à 70 % du poids total de la composition et plus préférentiellement de 20 % à 60 % en poids.

La composition de l'invention peut être à usage cosmétique ou pharmaceutique. Dans le cadre de l'invention revendiquée, elle est à usage cosmétique et non thérapeutique, et en particulier d'application topique sur la peau et les fibres kératiniques, et plus spécialement sur le cuir chevelu, les cheveux et les cils d'êtres humains. Aussi, la composition doit contenir un milieu physiologiquement acceptable non toxique et susceptible d'être appliqué sur la peau, y compris le cuir chevelu et les paupières, et les fibres kératiniques d'êtres humains.

Cette composition peut se présenter sous toutes formes galéniques connues adaptées au mode d'utilisation.

### Matières premières non essentielles à l'invention

### Forme galénigue :

Pour une application topique sur la peau, y compris le cuir chevelu, la composition peut avoir la forme d'une solution, d'une suspension, d'une émulsion ou dispersion de consistance plus ou moins fluide et notamment liquide ou semi-liquide, obtenue par dispersion d'une phase grasse dans une phase aqueuse (H/E) ou inversement (E/H), ou encore, d'un gel.

On peut également envisager une composition sous forme de mousse ou encore sous forme de spray ou d'aérosol comprenant alors un agent propulseur sous pression.

En particulier la composition à application sur le cuir chevelu ou les cheveux peut se présenter sous forme d'une lotion de soin capillaire, par exemple d'application journalière ou bihebdomadaire, d'un shampooing ou d'un après-shampooing capillaire, en particulier d'application bihebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Par ailleurs, pour une application sur les cils ou les poils, la composition à laquelle s'applique l'invention peut se présenter sous forme d'un mascara, pigmenté ou non, à appliquer à la brosse sur les cils ou encore sur les poils de barbe ou de moustache.

Selon un mode de réalisation particulier, la composition selon l'invention se présente sous forme de crème ou lotion capillaire, de shampooing ou d'après-shampooing capillaire, de mascara pour cheveux ou pour cils.

Les quantités des différents constituants du milieu physiologique de la composition selon l'invention sont ceux généralement utilisées dans les domaines considérés. En outre, ces compositions sont préparées selon les méthodes usuelles.

Lorsque la composition est une émulsion, la proportion de la phase grasse peut aller de 2 % à 80 % en poids, et de préférence de 5 % à 50 % en poids par rapport au poids total de la composition. La phase aqueuse est ajustée en fonction de la teneur en phase grasse, en dérivé de vitamine F et de glucose, en alcool et tensioactif non-ionique ainsi que de celle des éventuels ingrédients additionnels, pour obtenir 100% en poids. En pratique la phase aqueuse représente de 5 % à 89,9% en poids.

La phase grasse peut contenir des composés gras ou huileux, liquides à température ambiante (25°C) et pression atmosphérique (760 mm de Hg), généralement appelés huiles, des cires et des produits pâteux ou semi-solides. Ces huiles peuvent être compatibles entre elles et former une phase grasse liquide macroscopiquement homogène.

La phase aqueuse contient de l'eau et éventuellement un ingrédient miscible à l'eau comme les polyols tels que le propylène glycol, le glycérol ou le sorbitol.

### Additifs de formulation

La composition de l'invention peut comprendre, en outre, un ou plusieurs autres ingrédients usuellement utilisés dans les domaines concernés, choisis parmi les additifs de formulation comme les épaississants ou gélifiants de phase aqueuse ou de phase huileuse, les matières colorantes solubles dans le milieu de la composition, les particules solides du type charges ou pigments, minéraux ou organiques sous forme de micro ou nano-particules, les conservateurs, les parfums, les hydrotopes ou électrolytes, les neutralisants (alcalinisant ou acidifiant), les agents propulseurs, les tensioactifs anioniques, cationiques ou amphotères, les polymères en particulier filmogènes, anioniques, non ioniques, cationiques ou amphotères hydrosolubles ou hydrodispersibles, les sels minéraux ou organiques, les chélatants ; leurs mélanges. Ces additifs peuvent être présents dans la composition selon les quantités généralement utilisées dans le domaine cosmétique et dermatologique et notamment à raison de 0,01 à 50% du poids total de la composition et mieux de 0,1 à 20% et par exemple de 0,1 à 10 %.

Bien entendu l'homme du métier veillera à choisir les éventuels ingrédients complémentaires et/ou leur quantité de telle manière que les propriétés avantageuses de la composition selon l'invention, notamment à savoir l'augmentation de la densité des fibres kératiniques, ne soient pas ou substantiellement pas, altérées par l'adjonction envisagée.

Comme huiles utilisables dans l'invention, on peut citer les huiles d'origine minérale (huile de vaseline, isoparaffine hydrogénée), les huiles d'origine végétale (fraction liquide du beurre de karité, huile de tournesol, d'abricot, de soja, alcool ou acide gras), les huiles d'origine animale (perhydrosqualène), les huiles de synthèse (esters d'acide gras, huile de Purcellin), les huiles siliconées (polydiméthylsiloxanes linéaires ou cycliques, phényltriméthicones) et les huiles fluorées (perfluoropolyéthers). Comme cires, on peut citer les cires siliconées, les cires d'abeille, de candellila, de riz, de carnauba ou de paraffine ou de polyéthylène.

Lorsque la composition est sous forme d'émulsion, celle-ci contient, en outre, un ou plusieurs émulsionnants et éventuellement un ou plusieurs co-émulsionnants utilisés généralement dans les domaines cosmétique et pharmaceutique. Leur nature est, en outre, fonction du sens de l'émulsion. En pratique, l'émulsionnant et éventuellement le co-émulsionnant sont présents, dans la composition, en une proportion allant de 0,1 % à 30 % en poids, de préférence de 0,5 à 20 % en poids et mieux de 1 à 8%. L'émulsion peut, en outre, contenir des vésicules lipidiques et notamment des liposomes.

Comme épaississant ou gélifiant utilisable dans l'invention, on peut citer les polymères épaississants ou gélifiants comme les acides polyacryliques réticulés, les polymères associatifs et les épaississants ou gélifiants non polymériques comme les argiles modifiées ou non, les amides, les sels métalliques d'acides gras.

### Actifs cosmétiques et pharmaceutiques

La composition de l'invention peut comprendre, en outre, un ou plusieurs actifs cosmétiques et pharmaceutiques à action bénéfique pour la peau, et notamment pour le cuir chevelu, et les fibres kératiniques (autres que le dérivé de vitamine F et de glucose). Comme actif cosmétique ou pharmaceutique à action bénéfique pour les cheveux ou le cuir chevelu, utilisable dans l'invention, on peut citer le ou les actifs choisis parmi :
- les agents bloqueurs d'U.V. tels que les filtres solaires ;
- les vitamines (A, C, E) et leurs dérivés (palmitate de rétinol, acétate ou palmitate de tocophérol) ;
- les céramides ;
- les protéines et leurs hydrolysats, les peptides, les acides aminés (naturels) ;
- l'urée, l'allantoïne ;
- les sucres et les dérivés de sucre comme les sucres réduits ou oxydés ;
- les extraits d'origine végétale (ceux d'Iridacées ou de soja) ou bactérienne ;
- les hydroxy-acides en particulier les acides hydroxycarboxyliques ou cétocarboxyliques (acide de fruit, acide salicylique) et leurs esters comme le n-octanoyl-5 salicylique ;
- le diazoxyde, la spiroxasone, les phospholipides comme la lécithine notamment déshuilée;
- les composés actifs additionnels favorisant la repousse et/ou limitant la chute des cheveux. tels que, notamment : les esters d'acide nicotinique, dont notamment le nicotinate de tocophérol, le nicotinate de benzyle et les nicotinates d'alkyles en C₁-C₂₂ comme les nicotinates de méthyle ou d'hexyle ; les dérivés de pyrimidine, comme le 2,4-diamino 6-piperidinopyrimidine 3- oxyde ou « minoxidil » décrit dans les brevets US 4139619 et US 4592812 ; les dérivés de pyrimidine 3-oxyde comme ceux décrits dans WO 92/01437 ou WO 96/09048, et notamment l' « aminexil » ou 2,4-diaminopyrimidine 3-N-oxyde ; les antiandrogènes comme les inhibiteurs stéroïdiens ou non stéroïdiens de 5α-réductase comme le finastéride et les composés décrits dans US 5 516779, l'acétate de cyprostérone, l'acide azélaïque, ses sels et ses dérivés et les composés décrits dans US 5 480913, le flutamide et les composés décrits dans les brevets US 5 411981, 5 565467 et 4 910226 , les prostaglandines comme PGF2-a ou PGE2 sous forme de sel ou d'esters, leurs analogues comme le latanoprost ;
- les agents antibactériens ou antifongiques ou antipelliculaires comme les dérivés du sélénium, le kétoconazole, l'octopirox, le triclocarban, le triclosan, le pyrithione zinc, l'itraconazole, l'acide asiatique, l'hinokitiol, la mipirocine, les tétracyclines, notamment l'érythromycine et les composés décrits dans EP 0680745, le chlorhydrate de clinycine, le peroxyde de benzoyle ou de benzyle et la minocycline ;
- les agents antagonistes de canaux calcium et les agents agonistes de canaux potassiques ;
- les hormones ;
- les agents anti-inflammatoires stéroïdiens comme les glucocorticoïdes, les corticostéroïdes (par exemple : l'hydrocortisone) et anti-inflammatoires non stéroïdiens comme l'acide glycyrrhétinique et l'α-bisabolol, la benzydamine et les composés décrits dans EP 0770399, WO 94/06434 et FR 2268523 ;
- les agonistes des récepteurs de RXR des rétinoïdes et les antagonistes des rétinoïdes ;
- les agents anti-radicaux libres et les antioxydants comme le ButylHydroxyAnisole ou le ButylHydroxyToluène ;
- les antiséborrhéiques ;
- les antiparasitaires ;
- les antiviraux ;
- les antiprurigineux ;
- les caroténoïdes comme le β-carotène ;
- les lactones et leurs sels correspondants ;
- les acides gras essentiels comme les acides linoléique, eicosatétraénoique, les acides linolénique, et eicosatriénoïque ou leurs esters et amides ;
- les huiles essentielles ;
- les phénols et polyphénols comme les flavonoïdes ;
- leurs mélanges.

Selon un mode de réalisation préféré de l'invention, la composition contient, en outre un actif additionnel favorisant la repousse et/ou limitant la chute des cheveux et en particulier du minoxidil, de l'aminexil, du latanoprost ou leurs mélanges. Cet actif additionnel peut être présent en quantité efficace pour réduire la chute des cheveux et/ou limiter leur chute et/ou augmenter leur densité et par exemple à une teneur de 0,001 % à 10 % en poids, de préférence de 0,1 % à 5 % et mieux de 0,5 % à 3 %.

### EXEMPLES

### Démonstration

(a) On réalise les 4 compositions suivantes (en grammes) :

| Composition | A | B | C | D |
|---|---|---|---|---|
| Dérivé de vitamine F et de glucose* | 5 | 5 | 5 | 5 |
| PEG 40 hydrogenated Castor Oil | | 3 | | 3 |
| Ethanol | 24,6 | 24,6 | 42,4 | 42,4 |
| Eau | qsp 100 | qsp 100 | qsp 100 | qsp 100 |
| Aspect de la composition (T0) | laiteuse | limpide | limpide | limpide |
| Aspect de la composition (J0+1) | laiteuse | limpide | laiteuse | limpide |

| | | | | |
|---|---|---|---|---|
| * obtenu selon l'exemple 1 du document EP-A-1371658. | | | | |

Ces essais illustrent parfaitement le rôle du tensioactif non ionique sur la solubilité de l'agent actif.
(b) On applique, séparément, 1 ml des compositions B, C, D fraîchement préparées sur trois mèches de cheveux naturels de 2,5g.

A l'application, la mèche traitée avec la composition C est très rêche et se démêle difficilement. Les mèches traitées avec les compositions B et D sont douces et se démêlent facilement. Après séchage, le toucher et l'aspect des cheveux traités avec les compositions B et D sont meilleurs qu'avec la composition C. Ils ont un toucher lisse, ils se démêlent facilement sans développer d'électricité statique et sont brillants.

Ces essais illustrent l'effet bénéfique du tensioactif sur les propriétés cosmétiques des cheveux.

### Exemple de composition selon l'invention

On réalise un sérum ayant pour composition :

| | |
|---|---|
| Dérivé de vitamine F et de glucose* | 0,1 g |
| Aminexil | 1,5 g |
| PEG 40 Hydrogenated Castor Oil (HLB 13) | 0,1 g |
| Ethanol | 47,2 g |
| Eau | qsp100g |

La composition se présente sous forme d'une lotion fluide incolore parfaitement transparente.

Elle s'applique avec une grande facilité sur le cuir chevelu en se répartissant aisément. Elle sèche rapidement sans apporter d'inconfort. Les cheveux imprégnés par la composition sont doux et lisses.

## Revendications

1. Procédé de traitement non thérapeutique cosmétique des fibres kératiniques humaines et/ou du cuir chevelu **caractérisé par le fait qu'**il consiste à appliquer sur les fibres kératiniques humaines et/ou le cuir chevelu, une composition cosmétique physiologiquement acceptable comprenant :
(a) au moins un dérivé de glucose et de vitamine F,
(b) au moins un tensioactif non ionique de balance hydrophile/lipophile (HLB) supérieure à 10,
(c) au moins un alcool en C₁-C₄,
(d) de l'eau ;
à laisser celle-ci au contact de ces fibres kératiniques, et/ou du cuir chevelu et éventuellement à rincer ces fibres kératiniques et/ou le cuir chevelu.

2. Procédé selon la revendication 1, **caractérisée en ce que** le dérivé de glucose et de vitamine F est un dérivé O-acylé obtenu par estérification, partielle ou totale, de la vitamine F par le glucose.

3. Procédé -selon l'une des revendications précédentes, **caractérisée en ce que** le dérivé de glucose et de vitamine F est utilisé en une quantité représentant de 0,01 % à 10 % du poids total de la composition.

4. Procédé-selon l'une des revendications précédentes, **caractérisée en ce que** le dérivé de glucose et de vitamine F est utilisé en une quantité représentant de 0,1 % à 2 % du poids total de la composition.

5. Procédé-selon l'une des revendications précédentes, **caractérisée en ce que** le tensioactif non ionique de HLB supérieure à 10 est choisi parmi les alcools, les alpha-diols, les alkyl(C₁-C₂₀)phénols ou les acides gras polyéthoxylés, polypropoxylés ou polyglycérolés, les copolymères d'oxyde d'éthylène et de propylène, les condensats d'oxyde d'éthylène et de propylène sur des alcools gras ; les amides gras polyéthoxylés ; les amides gras polyglycérolés ; les esters d'acides gras du sorbitane éthoxylés ; les esters d'acides gras du saccharose ; les esters d'acides gras du polyéthylèneglycol ; les alkyl(C₆-C₂₄)polyglycosides ; les dérivés de N-alkyl(C₆-C₂₄)glucamine ; les oxydes d'amines ; et leurs mélanges.

6. Procédé selon l'une des revendications précédentes, **caractérisée en ce que** le tensioactif non ionique de HLB supérieure à 10 est choisi parmi les alcools, les alpha-diols, les alkyl(C₁-C₂₀)phénols ou les acides gras polyéthoxylés, polypropoxylés ou polyglycérolés, hydrogénés ayant une chaîne grasse comportant de 8 à 18 atomes de carbone, le nombre moyen de groupements d'oxyde d'éthylène ou d'oxyde de propylène allant de 3,5 à 200 et le nombre de groupements de glycérol allant de 2 à 100, leurs mélanges.

7. Procédé selon l'une des revendications précédentes, **caractérisée en ce que** le tensioactif non ionique ou le mélange de tensioactifs non-ioniques, de HLB supérieure à 10, est utilisé en quantité suffisante pour solubiliser avec l'alcool le dérivé de vitamine F et de glucose.

8. Procédé selon l'une des revendications précédentes, **caractérisée en ce que** le tensioactif non ionique ou le mélange de tensioactifs non-ioniques, de HLB supérieure à 10, est utilisé à une concentration allant de 0,01 % à 10 % du poids total de la composition.

9. Procédé- selon l'une des revendications précédentes, **caractérisée en ce que** le tensioactif non ionique ou le mélange de tensioactifs non-ioniques, de HLB supérieure à 10, est utilisé à une concentration allant de 0,1 % à 2 % du poids total de la composition.

10. Procédé selon l'une des revendications précédentes, **caractérisée en ce que** l'alcool en C₁-C₄ est choisi parmi l'éthanol ou l'isopropanol, leurs mélanges.

11. Procédé selon l'une des revendications précédentes, **caractérisée en ce que** l'alcool en C₁-C₄ ou le mélange de ces alcools est utilisé à une concentration de 2 % à 80% du poids total de la composition.

12. Procédé selon l'une des revendications précédentes, **caractérisée en ce que** l'alcool en C₁-C₄ ou le mélange de ces alcools est utilisé à une concentration de 20 % à 60 % du volume total de la composition.

13. Procédé selon l'une des revendications précédentes, **caractérisée en ce que** la composition se présente sous forme de crème ou lotion capillaire, de shampooing ou d'après-shampooing capillaire, de mascara pour cheveux ou pour cils.

14. Procédé selon l'une des revendications précédentes, **caractérisée en ce que** la composition comprend un ou plusieurs autres ingrédients choisis parmi les épaississants ou gélifiants de phase aqueuse ou de phase huileuse, les matières colorantes solubles dans le milieu de la composition, les charges et les pigments, minéraux ou organiques sous forme de micro ou nano-particules, les conservateurs, les parfums, les hydrotopes, les électrolytes, les neutralisants, les agents propulseurs, les tensioactifs anioniques, cationiques ou amphotères, les polymères filmogènes, anioniques, non ioniques, cationiques ou amphotères hydrosolubles ou hydrodispersibles, les sels minéraux ou organiques, les chélatants ; leurs mélanges.

15. Procédé selon l'une des revendications précédentes, **caractérisée en ce que** la composition comprend un ou plusieurs actifs cosmétiques ou pharmaceutiques à action bénéfique pour les cheveux ou le cuir chevelu choisis parmi les agents bloqueurs d'U.V. ; les vitamines et leurs dérivés ; les céramides ; les protéines, leurs hydrolysats, les peptides, les acides aminés ; l'urée, l'allantoïne ; les sucres et les dérivés de sucre ; les extraits d'origine végétale ou bactérienne ; les hydroxy-acides et leurs esters ; le diazoxyde, la spiroxasone, les phospholipides ; les esters d'acide nicotinique ; le minoxidil ; l'aminexil ; les inhibiteurs stéroïdiens ou non stéroïdiens de 5α-réductase ; les agents antibactériens, antifongiques ou antipelliculaires ; les agents antagonistes de canaux calcium ; les agents agonistes de canaux potassiques ; les hormones ; les agents anti-inflammatoires stéroïdiens et non stéroïdiens ; les agonistes des récepteurs de RXR des rétinoïdes et les antagonistes des rétinoïdes ; les agents anti-radicaux libres et les antioxydants ; les antiséborrhéiques ; les antiparasitaires ; les antiviraux ; les antiprurigineux ; les caroténoïdes ; les lactones et leurs sels correspondants ; les acides gras essentiels ou leurs esters et amides ; les huiles essentielles ; les phénols et polyphénols ; leurs mélanges.

16. Procédé selon l'une des revendications précédentes, **caractérisée en ce que** la composition comprend, en outre, un actif additionnel favorisant la repousse et/ou limitant la chute des cheveux.

17. Procédé selon l'une des revendications précédentes, **caractérisée en ce que** la composition comprend, en outre, un actif additionnel favorisant la repousse et/ou limitant la chute des cheveux choisi parmi le minoxidil, l'aminexil, le latanoprost ou leurs mélanges.

## Patentansprüche

1. Verfahren zur nicht-therapeutischen kosmetischen Behandlung menschlicher Keratinfasern und/oder Kopfhaut, **dadurch gekennzeichnet, dass** man bei dem Verfahren auf die menschlichen Keratinfasern und/oder die Kopfhaut eine physiologisch annehmbare Kosmetikzusammensetzung aufbringt, die Folgendes umfasst:
(a) mindestens ein Derivat von Glucose und Vitamin F,
(b) mindestens ein nichtionisches Tensid mit einer Hydrophil-Lipophil-Balance (HLB) von mehr als 10,
(c) mindestens einen C₁-C₄-Alkohol,
(d) Wasser,
man diese mit den Keratinfasern und/oder der Kopfhaut in Kontakt belässt und gegebenenfalls die Keratinfasern und/oder die Kopfhaut spült.

2. Verfahren nach Anspruch 1, **dadurch gekennzeichnet, dass** das Derivat von Glucose und Vitamin F ein 0-Acyl-Derivat ist, das durch partielle oder vollständige Veresterung von Vitamin F mit Glucose erhalten wird.

3. Verfahren nach einem der vorhergehenden Ansprüche, **dadurch gekennzeichnet, dass** das Derivat von Glucose und Vitamin F in einer Menge verwendet wird, die 0,01% bis 10%, bezogen auf das Gesamtgewicht der Zusammensetzung, ausmacht.

4. Verfahren nach einem der vorhergehenden Ansprüche, **dadurch gekennzeichnet, dass** das Derivat von Glucose und Vitamin F in einer Menge verwendet wird, die 0,1% bis 2%, bezogen auf das Gesamtgewicht der Zusammensetzung, ausmacht.

5. Verfahren nach einem der vorhergehenden Ansprüche, **dadurch gekennzeichnet, dass** das nichtionische Tensid mit einer HLB von mehr als 10 aus Alkoholen, alpha-Diolen, (C₁-C₂₀)Alkylphenolen oder polyethoxylierten, polypropoxylierten oder polyglycerinierten Fettsäuren, Copolymeren von Ethylen- und Propylenoxid, Kondensaten von Ethylen- und Propylenoxid mit Fettalkoholen; polyethoxylierten Fettsäureamiden, polyglycerinierten Fettsäureamiden, ethoxylierten Fettsäureestern von Sorbitan, Fettsäureestern von Saccharose, Fettsäureestern von Polyethylenglykol, (C₆-C₂₄) Alkylpolyglycosiden; Derivaten von N- (C₆-C₂₄)Alkylglucamin, Aminoxiden und deren Gemischen ausgewählt ist.

6. Verfahren nach einem der vorhergehenden Ansprüche, **dadurch gekennzeichnet, dass** das nichtionische Tensid mit einer HLB von mehr als 10 aus Alkoholen, alpha-Diolen, (C₁-C₂₀)Alkylphenolen oder polyethoxylierten, polypropoxylierten oder polyglycerinierten hydrierten Fettsäuren mit einer Fettkette, die 8 bis 18 Kohlenstoffatome enthält, wobei die durchschnittliche Anzahl von Ethylenoxid- oder Propylenoxidgruppen von 3,5 bis 200 beträgt und die Anzahl der Glyceringruppen von 2 bis 100 beträgt, deren Gemischen ausgewählt ist.

7. Verfahren nach einem der vorhergehenden Ansprüche, **dadurch gekennzeichnet, dass** das nichtionische Tensid oder das Gemisch von nichtionischen Tensiden mit einer HLB von mehr als 10 in einer Menge verwendet wird, die ausreicht, um das Derivat von Vitamin F und Glucose mit dem Alkohol zu solubilisieren.

8. Verfahren nach einem der vorhergehenden Ansprüche, **dadurch gekennzeichnet, dass** das nichtionische Tensid oder das Gemisch von nichtionischen Tensiden mit einer HLB von mehr als 10 in einer Konzentration von 0,01% bis 10%, bezogen auf das Gesamtgewicht der Zusammensetzung, verwendet wird.

9. Verfahren nach einem der vorhergehenden Ansprüche, **dadurch gekennzeichnet, dass** das nichtionische Tensid oder das Gemisch von nichtionischen Tensiden mit einer HLB von mehr als 10 in einer Konzentration von 0,1% bis 2%, bezogen auf das Gesamtgewicht der Zusammensetzung, verwendet wird.

10. Verfahren nach einem der vorhergehenden Ansprüche, **dadurch gekennzeichnet, dass** der C₁-C₄-Alkohol aus Ethanol oder Isopropanol, deren Gemischen ausgewählt ist.

11. Verfahren nach einem der vorhergehenden Ansprüche, **dadurch gekennzeichnet, dass** der C₁-C₄-Alkohol oder das Gemisch dieser Alkohole in einer Konzentration von 2% bis 80%, bezogen auf das Gesamtgewicht der Zusammensetzung, verwendet wird.

12. Verfahren nach einem der vorhergehenden Ansprüche, **dadurch gekennzeichnet, dass** der C₁-C₄-Alkohol oder das Gemisch dieser Alkohole in einer Konzentration von 20% bis 60%, bezogen auf das Gesamtvolumen der Zusammensetzung, verwendet wird.

13. Verfahren nach einem der vorhergehenden Ansprüche, **dadurch gekennzeichnet, dass** die Zusammensetzung in Form von Haarcreme oder -lotion, Shampoo oder Haarpflegespülung, Mascara für Haare oder für Wimpern vorliegt.

14. Verfahren nach einem der vorhergehenden Ansprüche, **dadurch gekennzeichnet, dass** die Zusammensetzung einen oder mehrere andere Inhaltsstoffe enthält, ausgewählt aus Verdickungsmitteln oder Gelbildnern für die wässrige Phase oder die Ölphase, in dem Medium der Zusammensetzung löslichen Farbstoffen, anorganischen oder organischen Füllstoffen und Pigmenten in Form von Mikro- oder Nanopartikeln, Konservierungsmitteln, Parfüms, Hydrotropen, Elektrolyten, Neutralisationsmitteln, Treibmitteln, anionischen, kationischen oder amphoteren Tensiden, filmbildenden anionischen, nichtionischen, kationischen oder amphoteren wasserlöslichen oder wasserdispergierbaren Polymeren, anorganischen oder organischen Salzen, Chelatbildungsmitteln, deren Gemischen.

15. Verfahren nach einem der vorhergehenden Ansprüche, **dadurch gekennzeichnet, dass** die Zusammensetzung einen oder mehrere kosmetische oder pharmazeutische Wirkstoffe mit einer vorteilhaften Wirkung auf die Haare oder die Kopfhaut umfasst, ausgewählt aus UV-Blockierungsmitteln, Vitaminen und deren Derivaten, Ceramiden, Proteinen, deren Hydrolysaten, Peptiden, Aminosäuren, Harnstoff, Allantoin, Zuckern und Zuckerderivaten, Extrakten pflanzlichen oder bakteriellen Ursprungs, Hydroxysäuren und deren Estern, Diazoxid, Spiroxason, Phospholipiden, Nicotinsäureestern, Minoxidil, Aminexil, steroidalen oder nichtsteroidalen 5α-Reduktase-Inhibitoren, antibakteriellen, fungiziden oder Antischuppenmitteln, Calciumkanalantagonisten, Kaliumkanalagonisten-Mitteln, Hormonen, steroidalen und nichtsteroidalen entzündungshemmenden Mitteln, Agonisten von RXR-Retinoidrezeptoren und Retinoidantagonisten, Mitteln gegen freie Radikale und Antioxidantien, Antiseborrhoika, Antiparasitenmitteln, Antivirenmitteln, Antipruritika, Carotinoiden, Lactonen und deren entsprechenden Salzen, essenziellen Fettsäuren oder deren Estern und Amiden, essenziellen Ölen, Phenolen und Polyphenolen, deren Gemischen.

16. Verfahren nach einem der vorhergehenden Ansprüche, **dadurch gekennzeichnet, dass** die Zusammensetzung außerdem einen zusätzlichen Wirkstoff umfasst, der das Nachwachsen der Haare fördert und/oder Haarausfall einschränkt.

17. Verfahren nach einem der vorhergehenden Ansprüche, **dadurch gekennzeichnet, dass** die Zusammensetzung außerdem einen zusätzlichen Wirkstoff umfasst, der das Nachwachsen der Haare fördert und/oder Haarausfall einschränkt, ausgewählt aus Minoxidil, Aminexil, Latanoprost oder deren Gemischen.

## Claims

1. Process for cosmetic non-therapeutic treatment of human keratin fibres and/or of the scalp, **characterized in that** it consists in applying, to the human keratin fibres and/or the scalp, a physiologically acceptable cosmetic composition comprising:
(a)at least one derivative of glucose and of vitamin F,
(b)at least one non-ionic surfactant with a hydrophilic/lipophilic balance (HLB) greater than 10,
(c)at least one C₁-C₄ alcohol,
(d)water;
in leaving said composition in contact with these keratin fibres and/or the scalp and optionally in rinsing these keratin fibres and/or the scalp.

2. Process according to Claim 1, **characterized in that** the derivative of glucose and of vitamin F is an 0-acylated derivative obtained by partial or total esterification of vitamin F with glucose.

3. Process according to either of the preceding claims, **characterized in that** the derivative of glucose and of vitamin F is used in an amount representing from 0.01% to 10% of the total weight of the composition.

4. Process according to one of the preceding claims, **characterized in that** the derivative of glucose and of vitamin F is used in an amount representing from 0.1% to 2% of the total weight of the composition.

5. Process according to one of the preceding claims, **characterized in that** that the non-ionic surfactant with an HLB greater than 10 is chosen from alcohols, alpha-diols, (C₁-C₂₀) alkyl phenols or polyethoxylated, polypropoxylated or polyglycerolated fatty acids, copolymers of ethylene oxide and of propylene oxide, condensates of ethylene oxide and of propylene oxide with fatty alcohols; polyethoxylated fatty amides; polyglycerolated fatty amides; ethoxylated fatty acid esters of sorbitan; fatty acid esters of sucrose; fatty acid esters of polyethylene glycol; (C₆-C₂₄)alkylpolyglycosides; N-(C₆-C₂₄)alkylglucamine derivatives; amine oxides; and mixtures thereof.

6. Process according to one of the preceding claims, **characterized in that** the non-ionic surfactant with an HLB greater than 10 is chosen from alcohols, alpha-diols, (C₁-C₂₀)alkyl phenols or polyethoxylated, polypropoxylated or polyglycerolated hydrogenated fatty acids which have a fatty chain comprising from 8 to 18 carbon atoms, the average number of ethylene oxide or propylene oxide groups ranging from 3.5 to 200 and the number of glycerol groups ranging from 2 to 100, and mixtures thereof.

7. Process according to one of the preceding claims, **characterized in that** the non-ionic surfactant or the mixture of non-ionic surfactants, with an HLB greater than 10, is used in an amount sufficient to dissolve with the alcohol the derivative of vitamin F and of glucose.

8. Process according to one of the preceding claims, **characterized in that** the non-ionic surfactant or the mixture of non-ionic surfactants, with an HLB greater than 10, is used at a concentration ranging from 0.01% to 10% of the total weight of the composition.

9. Process according to one of the preceding claims, **characterized in that** the non-ionic surfactant or the mixture of non-ionic surfactants, with an HLB greater than 10, is used at a concentration ranging from 0.1% to 2% of the total weight of the composition.

10. Process according to one of the preceding claims, **characterized in that** the C₁-C₄ alcohol is chosen from ethanol and isopropanol, and mixtures thereof.

11. Process according to one of the preceding claims, **characterized in that** the C₁-C₄ alcohol or the mixture of these alcohols is used at a concentration of from 2% to 80% of the total weight of the composition.

12. Process according to one of the preceding claims, **characterized in that** the C₁-C₄ alcohol or the mixture of these alcohols is used at a concentration of from 20% to 60% of the total volume of the composition.

13. Process according to one of the preceding claims, **characterized in that** the composition is in the form of a hair cream or lotion, a shampoo or a hair conditioner, or a hair or eyelash mascara.

14. Process according to one of the preceding claims, **characterized in that** the composition comprises one or more other ingredients chosen from aqueous-phase or oily-phase thickeners or gelling agents, colourants that are soluble in the medium of the composition, fillers and pigments, which are mineral or organic and in the form of microparticles or nanoparticles, preservatives, fragrances, hydrotopes, electrolytes, neutralizing agents, propellants, anionic, cationic or amphoteric surfactants, water-soluble or water-dispersible anionic, non-ionic, cationic or amphoteric film-forming polymers, mineral or organic salts, chelating agents; and mixtures thereof.

15. Process according to one of the preceding claims, **characterized in that** the composition comprises one or more cosmetic or pharmaceutical active agents with a beneficial action on the hair or the scalp, chosen from UV-blockers; vitamins and derivatives thereof; ceramides; proteins, hydrolysates thereof, peptides, amino acids; urea, allantoin; sugars and sugar derivatives; extracts of plant or bacterial origin; hydroxy acids and esters thereof; diazoxide, spiroxazone, phospholipids; nicotinic acid esters; minoxadil; aminexil; steroidal or non-steroidal 5α-reductase inhibitors; antibacterial, antifungal or antidandruff agents; calcium channel antagonists; potassium channel agonists; hormones; steroidal and non-steroidal anti-inflammatories; RXR retinoid receptor agonists and retinoid antagonists; free-radical scavengers and antioxidants; anti-seborrhoeic agents; anti-parasitic agents; antiviral agents; anti-pruritic agents; carotenoids; lactones and corresponding salts thereof; essential fatty acids or esters and amides thereof; essential oils; phenols and polyphenols; and mixtures thereof.

16. Process according to one of the preceding claims, **characterized in that** the composition also comprises an additional active agent which promotes hair regrowth and/or limits hair loss.

17. Process according to one of the preceding claims, **characterized in that** the composition also comprises an additional active agent which promotes hair regrowth and/or limits hair loss, chosen from minoxidil, aminexil, latanoprost or mixtures thereof.
